# EUROPEAN PATENT SPECIFICATION

(11) **EP 2 857 001 B1**
(45) Date of publication and mention of the grant of the patent: **09.09.2020**
(21) Application number: 13796284.1
(22) Date of filing: 27.05.2013
(51) Int. Cl.: A61K 8/19, A61K 8/02, A61K 8/365, A61K 8/86, A61Q 19/10

(54) **METHOD FOR PRODUCING TABLET, AND TABLET**
VERFAHREN ZUR HERSTELLUNG EINER TABLETTE UND TABLETTE
PROCÉDÉ DE FABRICATION D'UN COMPRIMÉ ET COMPRIMÉ

(30) Priority: 28.05.2012 JP 2012121078; 17.10.2012 JP 2012229900
(43) Date of publication of application: 08.04.2015
(73) Proprietor: Hot Album Tansansen Tablet, Inc., Hachiouji-shi, Tokyo 192-0082 (JP)
(72) Inventor: KOBOSHI, Shigeharu, Hachiouji-shi, Tokyo 192-0082 (JP); YOSHIMOTO, Hiroshi, Hachiouji-shi, Tokyo 192-0082 (JP)
(74) Representative: Carpmael, Robert Maurice Charles
(86) International application number: PCT/JP2013/064585
(87) International publication number: WO 2013/180048

(56) References cited:
- EP-A1- 0 378 172
- EP-A1- 1 707 197
- EP-A1- 2 189 154
- WO-A1-2010/144943
- WO-A1-2012/153383
- JP-A- H02 275 714
- JP-A- H09 286 718
- JP-A- 2002 241 261
- JP-A- 2004 083 584
- JP-A- 2011 105 615
- JP-A- 2011 190 189
- JP-A- 2013 129 654
- US-A1- 2005 019 412
- US-B1- 6 316 029

## Description

### FIELD OF THE INVENTION

The present invention relates to a bathing tablet manufacturing method and a bathing tablet. More specifically, the present invention relates to a bathing tablet manufacturing method and a bathing tablet composed of carbon dioxide gas bubble-forming compositions in which bicarbonate and an organic acid are mixed at a certain ratio to provide a tablet higher in hardness than a certain hardness, as a carbon dioxide gas bathing agent, and where the tablet is dissolved in hot water to be used (which includes water, heated or humidified hot water and a mixture of them in the present invention), an environment is developed that although an aqueous solution thereof immediately after dissolution is in a pH range of 5.5 to 8.5, bubbles of carbon dioxide gas are generated intensively and continuously from the tablet high in hardness, bubbles of carbon dioxide gas can be generated to give microsize fine bubbles smaller in diameter than a certain diameter, the bubbles are increased in surface area to dramatically enhance the dissolution degree of carbon dioxide gas in hot water, bubbles are generated slowly for a prolonged period of time inside the tablet to give uniform and small-sized bubbles, with a small quantity of the bubbles joined together, carbon dioxide gas components on the surface of bubbles are sufficiently dissolved in water or hot water, the hot water becomes neutral in pH after dissolution, thereby carbon dioxide gas changes to bicarbonate ions, providing a highly concentrated bicarbonate spring, and absorption of bicarbonate ions through the skin is made maximum to facilitate dissolution in the blood and increase concentrations of bicarbonate ions, thereby exhibiting great health promoting effects such as improvement of blood circulation at the time of bathing and elevation of body temperature.

### BACKGROUND AND DESCRIPTION OF RELATED ART

A mixture which contains bicarbonate (sodium hydrogen carbonate or potassium hydrogen carbonate) and an organic acid is molded by making tablets or others, thereby providing a bubble-forming composition (solid substance) by utilizing neutralization reactions. And, this process has been applied to products such as a cleaning agent, a bath liquid, a bathwater detergent and a pool-water disinfectant. These products (solid substances) are advantageous in that they will readily dissolve when placed into hot water, while generating carbon dioxide gas through neutralization reactions of sodium bicarbonate with an organic acid and also effective in enhancing a commercial value as they impart a comfortable usability to consumers. In particular, in bath liquids, it is said that the thus generated carbon dioxide gas is absorbed through the skin into blood vessels to provide effects of improving blood circulation, and the effects have been actively utilized.

However, where bicarbonate is combined with an organic acid to make a tablet and the tablet is dissolved in hot water, neutralization reactions take place intensively during dissolution to generate bubbles of carbon dioxide gas large in diameter. The bubbles are joined together to increase dimensionally, a buoyant force thereof allows carbon dioxide gas to rise quickly, being released into the air outside liquid, thereby carbon dioxide gas dissolved in hot water is decreased in concentration to lower the effects of bathing by carbon dioxide gas, which poses a problem.

Inherently, carbon dioxide gas is difficult to dissolve in hot water and further decreased in dissolution degree with an increase in temperature. And, concentrations of carbon dioxide gas in hot water are decreased without limit. Therefore, highly concentrated carbonated water was not possible unless carbon dioxide gas was compressed for dissolution under a high pressure by pressurization, as carried out in the production of carbonated beverages.

The inventors have found that although the force of bubbles coming from generation of bubbles can be enjoyed, carbon dioxide gas is actually low in concentration in hot water and carbonic acid is dissolved into blood vessels in a small quantity by absorption through the skin, thus resulting in a failure of obtaining the effects of bathing such as warming the body, which poses a problem.

It is said that a naturally occurring carbonated spring is required to have carbon dioxide gas at concentrations of 1000ppm or more. This spring is such that carbon dioxide gas is dissolved at high concentrations under ground at a high pressure. In an attempt to realize an artificial carbonated spring with carbon dioxide gas at concentrations of 1000ppm, carbon dioxide gas is required to be dissolved by adopting special equipment such as a highpressure gas cylinder and a membrane. The equipment is high in cost and large in size, resulting in equipment which is not used simply and easily.

Under these circumstances, as a method for utilizing a carbonated spring at home simply and easily, a bath liquid which will dissolve easily while generating carbon dioxide gas by just being placed in a bath has been widely accepted. And, a bath liquid which uses bicarbonate and an organic acid to generate bubbles through neutralization reactions (sometimes referred to as a bathing agent in the present invention) has become predominant in use.

In this case, a carbonated spring which develops a phenomenon that carbon dioxide gas is produced in a great quantity and bubbles are adhered on the skin is commonly regarded as a good carbonate spring. In general, the carbonated spring is designed so as to be weakly acidic at a pH value of 4 or more but less than 5.5 and an organic acid which is excessively acidic is added and mixed to cause neutralization reactions intensively, thereby dissolving bubble-forming carbon dioxide gas into hot water.

According to the study made by the present inventors, as described previously, the method causes intense neutralization reactions under weakly acidic conditions, bubbles of carbon dioxide gas are increased in diameter almost endlessly, carbon dioxide gas is mostly joined together and increased in size and escapes into the air outside liquid due to a great buoyant force. And carbon dioxide gas is apparently generated in a great quantity but carbonate which has been actually dissolved in hot water is not increased in concentrations, giving concentrations of only about 100ppm or less much lower than the above-described concentrations of 1000ppm.

In addition to the fact that most of naturally occurring carbonated springs are weakly acidic, there is a misleading common belief that a carbonated spring is such that concentrations of carbon dioxide gas are 1000ppm or more. Accordingly, it has been considered that anything is acceptable as long as carbon dioxide gas is high in concentration. As a result, a general practice has been conducted that a bathing agent is used to cause intense neutralization reactions under acidic conditions so that bubbles adhere on the skin. It has been, however, found that where a bath liquid is acidic, carbon dioxide gas is likely to be vaporized into the air as a gas outside liquid and, therefore, a first user can enjoy generation of bubbles while bathing and also enjoy some body warming effects due to salt concentrations, etc., and, then, where another family member takes a bath after generation of bubbles, the family member is not able to enjoy effects of warming the body because carbon dioxide gas hardly remains in the liquid, and it is required to add a new bathing agent for every bathing.

The present inventors have found that carbon dioxide gas is accordingly generated in hot water under reaction conditions that carbon dioxide gas can be easily dissolved in liquid and in order that carbon dioxide gas is absorbed through the skin to blood vessels, a bath liquid is required to be kept neutral which is close to a pH value of body fluid as much as possible

That is, on the basis of detailed evaluation of the effects of a naturally occurring carbonated spring made by the present inventors, it has been found that the naturally occurring carbonated spring is weakly acidic only in terms of a pH value which is in a weakly acidic range and not due to acidic components, a trace amount of mineral ions is dissolved deep underground at a high pressure, the trace amount of minus ions is dissociated to yield weak acidity free of potency (acidity not derived from excess of an organic acid), or weak acidity which is an extent that carbon dioxide gas is neutralized and becomes neutral only by coming into contact
with body fluid adhering to skin or hair such as sweat or lipid, and a commercially available bathing agent is quite meaningless in that only a pH value is brought closer to a pH value of a naturally occurring carbonated spring so that an organic acid is excessively added to be weakly acidic for easy generation of bubbles of carbon dioxide gas.

In general, in explaining the effects of a carbonated spring, it is stated that carbon dioxide gas is directly absorbed through the skin, but this is totally wrong. There is a contradiction that if carbon dioxide gas is directly absorbed through the skin and dissolved into blood vessels, why is carbon dioxide gas in the air not absorbed through the skin.

Further, blood and body fluid on the surface of the skin are almost neutral and in a pH range of 7.2 to 7.4. If the body fluid is neutral, carbonate components are present only as bicarbonate ions chemically, and components which are absorbed through the skin must be bicarbonate ions.

That is, the components are dissolved not as carbon dioxide gas but as bicarbonate ions in blood vessels from estimation of the pH. This is because a scientifically correct understanding is that carbonate ions are present in a different way, in other words, available as carbonate under weakly acidic conditions, as bicarbonate ions HCO₃ minus 1 ion under neutral conditions and as carbonate ion CO₃ minus 2 ions under alkaline conditions and carbonate ions are always dissolved as bicarbonate ions in body fluid which is neutral.

As described previously, a correct explanation is that a naturally occurring carbonated spring is weakly acidic only in terms of pH and it is quite free of potency and CO₂ (carbon dioxide gas) adhered on the skin is immediately neutralized, absorbed as bicarbonate ions through the skin and dissolved in capillary vessels.

On the assumption that a bathing agent is in a state of acidity having potency, that is, in excess of an organic acid, even in the case of excessive generation of bubbles of carbon dioxide gas, the carbon dioxide gas is very likely to be vaporized into the air and if the gas adheres on the skin and body fluid in a great quantity, there is no possibility that the gas is neutralized by the body fluid and not changed into bicarbonate ions. Therefore, the carbon dioxide gas is not absorbed into blood vessels or not able to provide health promoting effects such as enhancement of blood circulation and elevation of body temperature.

Thus, no health promoting effects are obtained by a conventional common sense of bathing agents that only generation of bubbles of carbon dioxide gas may be sufficient. In most cases, users are influenced by a wrong explanation that carbon dioxide gas is absorbed through the skin. As a result, research has concentrated on a combination of sodium bicarbonate with excess of an organic acid to realize only neutralization reactions and generation of bubbles, thus resulting in the design of bath liquids which are devoid of effects.

There has been a problem that despite great generation of bubbles of acid gas, carbon dioxide gas itself will not be absorbed through the skin but taken into blood vessels of the body, thereby resulting in a failure of obtaining the effects of bathing that a naturally occurring carbonated spring has, that is, blood circulation is facilitated to elevate body temperature.

Nevertheless, even if an organic acid is decreased in quantity to design a tablet or a bathing agent in a neutral or weakly alkaline state, research conducted by the present inventors has demonstrated that sufficient neutralization reactions will not actually take place, and the tablet or the bathing agent generates bubbles in an extremely subtle manner, thereby resulting in a failure of generating bubbles of carbon dioxide gas. This is also a meaningless issue.

The above-described bathing agent becomes a bathing liquid which only brings effects as a soap in which sodium bicarbonate is merely dissolved in hot water. The present inventors have found it necessary to design a bathing agent (tablet) which is quite difficult theoretically in that hot water becomes neutral after dissolution of the tablet but an environment that neutralization reactions take place intensively inside the tablet when placed into hot water, is developed, thereby generating fine microsize carbon dioxide gas, after completion of bubble generating reactions, the hot water becomes neutral or weakly alkaline. And, the inventors have tried to solve this difficult problem.

In other words, the present inventors have finally found that a bathing agent in which a reaction site is acidic or weakly acidic so as to generate carbon dioxide gas in a great quantity when placed into hot water, thereby developing an environment that generates a great quantity of carbon dioxide gas in liquid, is required and a pH of the hot water becomes neutral after dissolution of the bathing agent and that it is ideal to realize a bathing agent which gives to hot water a pH that the thus generated carbon dioxide gas is neutralized by body fluid when coming into contact with the skin or the body fluid and absorbed as bicarbonate ions through the skin.

Further, the more intense, the neutralization reactions become, the better the results become. Where bubbles are generated too quickly, they are increased in diameter, joined together and further increased in diameter to have a great buoyant force. As a result, most of the gas explodes on the surface of hot water and escapes into the air. As a result, carbon dioxide gas is dissolved in hot water to a lesser extent, and when bubbles are further increased in diameter, they are decreased in surface area. In this case as well, carbon dioxide gas is dissolved in hot water to a lesser extent.

It is, therefore, desirable that although bubbles are generated intensively, they are not joined together and generated continuously and slowly so as to be released for a prolonged period of time and fine microsize bubbles are generated stably in a great quantity. A method that polyethylene glycol or the like is simply used in a great quantity to prevent abrupt contact with reaction products, while the reaction products are allowed to come into contact slowly and continuously, thereby controlling reactions, has been so far proposed. However, in this case, neutralization reactions are also weakened to generate no bubbles, thus resulting in loss of effects of bathing. And, this poses a problem.

Therefore, existing bathing agents are mostly designed so that they are allowed to react intensively under weakly acidic conditions, with priority given to bubble generating reactions and, therefore, designed so as to show visually high concentrations of carbon dioxide gas and a wrong explanation is given that carbon dioxide gas is directly absorbed through the skin. These bathing agents are made into carbonated spring bathing agents in which although carbon dioxide gas is generated in a great quantity, the gas is instantly vaporized outside liquid, thus resulting in a failure of providing health promoting effects such as body warming effects by improvement in blood circulation due to absorption through the skin.

As a result, the above-described commercially available products are actually quite different from bathing agents which have the effects of bathing close to those of naturally occurring carbonated springs.

Incorrect knowledge or a wrong explanation that "carbon dioxide gas is directly absorbed through the skin" which will not, however, actually take place, has been widely accepted. Therefore, no attempt to develop new products for solving the above-described situation has been so far made at all. As a result, any one of commercially available bathing agents is unable to warm the body in practice, with an emphasis not being placed on essentially important components of a carbonate bathing agent but actively placed on non-essential parts of aroma or flavor thereof. As a matter of fact, the genuine value of bathing agents is neglected. Bicarbonate is combined with citric acid, and this should be the best combination in providing a tablet of a health-oriented bathing agent. However, even water which has been crystallized inside the tablet is also subjected to bubble generating reactions, and a sealed packaging container of the tablet swells out while being stored or delivered as a commercial product. And, this tablet has not been successfully developed as a commercial product. Actually, the tablet is designed in a manner that succinic acid or fumaric acid which is less likely to conduct neutralization reactions with bicarbonate is used to be excessively acidic, by which the tablet is not reactive on storage but will react intensively when placed into hot water. Or, a method of manufacturing the tablet has only been studied.

Conventionally, as a first method, a method in which double salt composed of carbonate and Glauber's salt is prepared in advance and an organic acid is mixed therewith and prepared, has been proposed (Patent Document 1).

As a second method, a method in which polyethylene glycol (hereinafter, sometimes abbreviated as "PEG") with an average molecular weight of 950 to 3700 at 30 to 70% by mass is formulated with other foamable components at 70 to 30% by mass and, thereafter, a resultant mixture is heated to melt PEG, by which the foamable components are inserted into PEG, has been proposed (Patent Document 2).

However, in the above-described methods for covering the components by using a great quantity of PEG, the components are mixed in a great quantity for making a product stable, and carbon dioxide gas is generated accordingly in a smaller quantity. Thus, a comfortable feeling of a consumer from use is lost and also active components for realizing an aim of the product are formulated in a smaller quantity. Therefore, carbon dioxide gas is generated in a smaller quantity and the product is used in a greater quantity in each use. Further, the individual components are not dissolved through neutralization reactions but dissolved independently. And, carbon dioxide gas is generated in a smaller quantity and, as a result, intended dissolution of carbon dioxide gas is not attained unless the product is used in a great quantity, which will eventually raise costs. Still further, a bathing agent which claims the effects resulting from generation of carbon dioxide gas is deprived of a commercial value, and this will be a fatal disadvantage.

On the other hand, in terms of productivity, in particular, in the case of tablets made by a tablet machine, these tablets are not given a mechanical strength and powder adheres on a mill and a rod of the tablet machine, which poses a problem. A binding agent (also referred to as a binder) and a mold release agent are used in a great quantity, which are also partially responsible for a decreased quantity of carbon dioxide gas to be generated. There is also a fear that fine powders of metallic soap which is generally used as a mold release agent are insoluble in water and may give an uncomfortable feeling from use.

In order to solve the above-described problems, a tablet manufacturing method in which an organic acid which is substantially free of water or will not liberate crystal water at 50°C or lower is heated, melted and mixed together with PEG at temperatures of 60°C to 100°C, thereafter, a resultant mixture thereof is cooled and converted to powder while being agitated by using an air-operated fluidized bed which is internally equipped with a paddle- or propeller-like agitation blade to which sodium hydrogen carbonate and sodium carbonate are then added, and the resultant is molded into a tablet, has been proposed. The organic acid which is substantially free of water or which will not liberate crystal water at 50°C or lower includes fumaric acid, tartaric acid, oxalic acid, citric acid, succinic acid, gluconic acid and adipic acid (Patent Document 3).

EP 1707197 A1 discloses a composition for the treatment of elevated levels of triglycerides.

US 2005/019412 A1 discloses nanoparticulate compositions comprising glipizide.

WO 2010/144943 A1 discloses buccal and/or sublingual formulation comprising one or more active compounds.

EP 2189154 A1 discloses pharmaceutical or nutraceutical composition in form of effervescent tablets or mouth-soluble granulates.

US 6316029 B1 discloses a rapidly disintegrating solid oral dosage form of a poorly soluble active ingredient and at least one pharmaceutically acceptable water-soluble or water-dispersible excipient.

EP 0378172 A1 discloses surface-treated sodium bicarbonate particles.

JP 2002 241261 A discloses a solid composition for a shower obtained by processing an inorganic salt and an optional ingredient for formulation into a solid granular and/or an agglomeratic composition for the shower.

### PRIOR ART DOCUMENTS

### PATENT DOCUMENTS

Patent Document 1: Japan Patent Pre-Publication No. S58-213714
Patent Document 2: Japan Patent Pre-Publication No. S58-105910
Patent Document 3: Japan Patent Pre-Publication No. H7-47532

### SUMMARY OF THE INVENTION

### PROBLEMS TO BE SOLVED BY THE PRESENT INVENTION

Therefore, a first object of the present invention is to provide a bathing tablet manufacturing method and a bathing tablet in which as a compound of a source for generating carbon dioxide gas and also as a compound of conducting neutralization reactions with bicarbonate (sodium hydrogen carbonate or potassium hydrogen carbonate), an organic acid is used and also in the presence of polyethylene glycol, compression molding is conducted to make a tablet higher in hardness than a certain hardness and larger in size than a certain size, thereby although an aqueous solution after dissolution of the tablet becomes neutral in pH, neutralization reactions are allowed to take place intensively and efficiently inside the tablet, bubbles of carbon dioxide gas which are made small in size as much as possible are continuously released for a certain period of time, by which the thus generated carbon dioxide gas is mostly dissolved in water without escaping into the air, and the aqueous solution immediately after dissolution is designed to become neutral in pH, bicarbonate ions in water are accordingly increased in concentration, carbon dioxide gas in contact with the skin is easily changed to bicarbonate ions due to a pH of the aqueous solution and the bicarbonate ions can be increased in concentration together with bicarbonate ions which are inherently available and absorbed into blood vessels through the skin as much as possible.

A second object of the present invention is to provide a method for manufacturing a bathing tablet such as a carbonated spring bathing agent in which dissolved carbon dioxide gas components which are high in concentration are easily neutralized with bicarbonate ions to raise concentrations of the bicarbonate ions absorbed through the skin as much as possible, blood circulation is remarkably facilitated to elevate body temperature, thereby enhancing the effects of bathing on health and beauty, and also to provide the tablet thereof.

Other objects of the present invention will be clarified by the following description.

In addition, in the present invention, "quantity" represents "quantity by mass," and "%" represents "% by mass," unless otherwise specified.

### MEANS FOR SOLVING THE PROBLEMS

The present invention which solves the above-described problems is arranged as follows.
[Clause 1] This is a bathing tablet manufacturing method comprising compression molding an organic acid in the presence of polyethylene glycol, an anhydride, and a bicarbonate to achieve the bathing tablet,
   wherein the organic acid is citric acid and optionally at least one selected from the group consisting of succinic acid, fumaric acid and malic acid, the anhydride is at least one selected from the group consisting of anhydrous sodium carbonate and anhydrous potassium carbonate, and the bicarbonate is at least one selected from the group consisting of sodium hydrogen carbonate and potassium hydrogen carbonate,
   wherein the ratio of the organic acid to the bicarbonate is in the range of 1:10 to 1:3, the ratio of the anhydride to the bicarbonate is in the range of 1:100 to 1:10, and the ratio of the polyethylene glycol to the bicarbonate is in the range of 1:100 to 1:5,
   wherein an aqueous solution immediately after dissolution of the bathing tablet has a pH of 5.5 to 8.5, and
   wherein the bathing tablet has a hardness of 25 kg or more, a diameter of 7mm or more, and a thickness of 7mm or more.
[Clause 2] This is the bathing tablet manufacturing method according to the above-described Clause 1 in which at least one of the bicarbonate and the organic acid is a granulated substance which is mixed with polyethylene glycol and granulated by using a fluidized-bed granulating machine.
[Clause 3] This is a bathing tablet which is obtained by subjecting an organic acid to compression molding in the presence of polyethylene glycol, an anhydride, and a bicarbonate,
   wherein the organic acid is citric acid and optionally at least one selected from the group consisting of succinic acid, fumaric acid and malic acid, the anhydride is at least one selected from the group consisting of anhydrous sodium carbonate and anhydrous potassium carbonate, and the bicarbonate is at least one selected from the group consisting of sodium hydrogen carbonate and potassium hydrogen carbonate,
   wherein the ratio of the organic acid to the bicarbonate is in the range of 1:10 to 1:3, the ratio of the anhydride to the bicarbonate is in the range of 1:100 to 1:10, and the ratio of the polyethylene glycol to the bicarbonate is in the range of 1:100 to 1:5,
   wherein an aqueous solution immediately after dissolution of the bathing tablet has a pH of 5.5 to 8.5, and
   wherein the bathing tablet has a hardness of 25 kg or more, a diameter of 7mm or more, and a thickness of 7mm or more.
[Clause 4] This is the bathing tablet according to the above-described Clause 3, in which an aqueous solution immediately after dissolution of the tablet is from 6.0 to 8.0 in pH.
[Clause 5] This is the bathing tablet according to the above-described Clause 3, which contains at least one of sodium n-(normal) octane sulfonate, sodium lauryl sulfonate, sodium lauroyl sarcosinate and myristoyl sodium methylalanine.

### Effects of the Invention

According to the above-described Clause 1 and Clauses 3 and 4, it is possible to generate continuously and slowly microsize bubbles of carbon dioxide gas with a certain diameter or less efficiently and for a prolonged period of time. It is also possible to adjust pH of an aqueous solution after dissolution of the tablet to a value which allows carbon dioxide gas to be neutralized easily into bicarbonate ions and dissolve at high concentrations. Further, the tablet is not damaged for neutralization reaction properties to keep sufficient concentrations of carbon dioxide gas, thereby enhancing absorption of carbon dioxide gas through the skin due to affinity of an organic acid such as citric acid with the skin. It is, therefore, possible to provide a carbonated spring bathing agent which is high in health and beauty promoting effects.

Further, according to the above-described Clauses 1 and 3, the tablet is made higher in hardness than a certain hardness and larger in size than a certain diameter. Thereby, neutralization reactions take place easily inside the tablet and microsize fine bubbles of carbon dioxide gas are generated efficiently inside the tablet to provide very small size bubbles continuously for a prolonged period of time. It is, thus, possible to dissolve carbon dioxide gas components dissolved in water at maximum concentrations and to provide remarkable effects of the present invention.

Further, according to the above-described Clause 1, at least one of the organic acids is citric acid. It is, therefore, possible to effect neutralization reactions efficiently even under conditions that an organic acid which becomes neutral after dissolution is available in a small quantity. Then, microsize bubbles of carbon dioxide gas can be generated efficiently and continuously to further increase the concentrations of carbon dioxide gas components dissolved in water, thereby providing the remarkable effects of the present invention.

Still further, according to the above-described Clause 1, an anhydride selected from anhydrous sodium carbonate and anhydrous potassium carbonate is added wherein the ratio of the organic acid to the bicarbonate is in the range of 1:10 to 1:3, the ratio of the anhydride to the bicarbonate is in the range of 1:100 to 1:10, and the ratio of the polyethylene glycol to the bicarbonate is in the range of 1:100 to 1:5. Thereby, polyethylene glycol can be added in a decreased quantity to enhance neutralization reaction properties, and the thus generated carbon dioxide gas is decreased further in microsize diameter to produce gas continuously and slowly for a prolonged period of time, thereby obtaining remarkable effects that carbon dioxide gas is dissolved in water in a greater quantity.

In addition, according to the above-described Clause 5, the tablet can be made into a harder tablet which will react favorably. Thus, it is possible to effect neutralization reactions more effectively and also to generate fine carbon dioxide gas, with clarity of hot water after dissolution kept, thereby providing more remarkable effects of the present invention. In addition, the tablet of the present invention is not restricted to applications such as a bath liquid and others described in Background Art. The tablet may be used in water for showers by being loaded into a shower head, for example.

### MODE FOR CARRYING OUT THE INVENTION

Hereinafter, a more detailed description will be given of the present invention.

In the present invention, even where an aqueous solution immediately after dissolution of the tablet is neutral in pH, an environment that neutralization reactions take place efficiently in the tablet which is higher in hardness and larger in size than a certain size, is developed. The reactions take place continuously for a certain period of time, by which carbon dioxide gas dissolved in water is sufficiently increased in concentration, thus making it possible to change dissolved carbonate components into bicarbonate ions with high concentrations.

In the present invention, it is important that pH immediately after dissolution of the tablet is in a range of 5.5 to 8.5 so that the thus dissolved carbon dioxide gas components are neutralized and dissolved as bicarbonate ions at high concentrations. It is also important that a pH range is preferably from 6.0 to 8.0 in view of generation of carbon dioxide gas and efficient conversion to bicarbonate.

And, pH immediately after dissolution has the meaning that the tablet generates spontaneously carbon dioxide gas immediately after dissolution, although bicarbonate ions are substantially neutral in pH, and pH will rise gradually. Although pH is, for example, 7.0, immediately after dissolution, pH is changed to about 7.5 in 24 hours. And, pH will rise further when air is blown by a jet bath or the like. Therefore, it is appropriate that pH after dissolution is defined by pH immediately after dissolution.

When used for washing the face, footbath or bathing, the tablet is required to be used in a quantity of 20g to 100g per 200 liters (0.01% to 5%). In this case, pH immediately after dissolution of the bathing agent is a pH value that is necessary for allowing bicarbonate ions to be available at high concentrations.

The tablet of the present invention is such that an acidic or alkaline pH adjusting agent is used whenever necessary to attain a pH value of the present invention. Therefore, the tablet of the present invention is able to effect neutralization reactions efficiently, generating carbon dioxide gas with an appropriate diameter for being dissolved in hot water at a suitable speed, and making neutral pH of the hot water or the aqueous solution after complete dissolution of the tablet.

In principle, no neutralization reactions will take place in neutrality. And, in the tablet, bicarbonate comes into contact with an organic acid at high concentrations, pH of an aqueous solution can be made weakly alkaline from neutral in a great quantity of dissolved water while effecting neutralization reactions. It has been found that the tablet is required to be higher in hardness and of a certain size in this sense as well.

The thus developed carbonate components are neutralized and changed into bicarbonate ions. On the assumption that from the beginning, sodium bicarbonate is fed in a powdery form to produce bicarbonate ions, no effects of bathing such that the body is warmed can be obtained. Thus, the inventors take pride in the remarkable fact of finding that no health and beauty promoting effects can be obtained, unless a system that carbon dioxide gas is generated to produce bicarbonate ions by way of the carbon dioxide gas as with a naturally occurring carbonated spring, is provided.

As described above, sodium bicarbonate and citric acid are allowed to react slowly and elaborately in the tablet high in hardness. It is, thereby, possible to obtain great effects of bathing as described in the present invention and to provide a commercial product having a high added value.

The above-described effects of the present invention are exhibited to a maximum extent by procedures in which an organic acid and polyethylene glycol are specified for the quantities in relation to bicarbonate and mixed respectively at certain ratios, and the tablet is designed so that pH of an aqueous solution immediately after dissolution of the tablet is within a certain range, when the tablet is subjected to compression molding so as to be higher in hardness than a certain hardness and larger in size than a certain size, water penetrates into the tablet to cause continuously intense and uniform reactions inside the tablet, generating bubbles of carbon dioxide gas as microsize fine carbonate gas, the tablet generates small and fine bubbles in a great quantity in a state of remaining firmly on the bottom of a bathtub until the end, the tablet will dissolve in water before floating up because of no buoyant force and a large surface area, thereby inevitably increasing the concentrations of carbon dioxide gas in liquid, and the tablet is prepared so that pH of water in which carbon dioxide gas is dissolved to give bicarbonate ions is adjusted in a range of 5.5 to 8.5.

Further, in the present invention, a mixture A of bicarbonate is a granulated substance which is prepared by being coated with polyethylene glycol using a fluidized bed. Therefore, the effects of the present invention such as uniform reactions of the tablet are greatly exhibited.

Still further, in the present invention, it is possible to carry out neutralization reactions inside the tablet at a maximum efficiency despite the fact that the higher the hardness of the tablet is, the more greatly, the aqueous solution becomes neutral.

At this time, the efficiency of neutralization reactions will be understood as follows. For example, even where bicarbonate soda (sodium bicarbonate) is mixed with citric acid at a ratio specified by the present invention, a tablet thereof which has been placed into a bath in a powdery form as it is will dissolve quickly within a few seconds, thereby generating only a small quantity of carbon dioxide gas. A step that individual components are dissolved and diluted before generation of carbon dioxide gas in neutralization reactions is developed instantly and preferentially, carbon dioxide gas is hardly dissolved in liquid, thereby yielding a thin neutral solution in which bicarbonate soda and citric acid soda are merely dissolved. Further, even upon generation of carbon dioxide gas, the gas is light in weight and reacts only on the surface due to a buoyant force. Thereby, carbon dioxide gas is hardly dissolved in the liquid of the bath but escapes into the air.

Commercially-available carbon dioxide gas bath agents are mostly those as described above. These bath liquids are low in hardness and small in size and will soon float on the surface of liquid. They are, therefore, not able to generate carbon dioxide gas efficiently or dissolve the gas into the liquid.

Where hot water becomes acidic after dissolution of a bathing agent, reactions proceed intensively and rapidly, thereby generating carbon dioxide gas explosively. Thus, a tablet thereof will degrade and dissolve completely while floating on the surface of the hot water and, then, carbon dioxide gas is substantially vaporized into the air. Further, even if dissolved carbonate gas still remains, the solution is weakly acidic, with pH being less than 5.5, and carbon dioxide gas floats up to the surface repeatedly as bubbles, vaporizing into the air and disappears. This is a disadvantage.

Therefore, in the present invention, bicarbonate is combined at a certain ratio with an organic acid, and a resultant thereof is subjected to compression molding to provide a tablet which is higher in hardness than a certain hardness. The tablet is preferably 7mm or more both in diameter and thickness and more preferably 10mm or more both in diameter and thickness. Then, when the tablet is dissolved in hot water, neutralization reactions can be carried out continuously and efficiently to generate fine and uniform bubbles, and carbon dioxide gas can be dissolved into hot water efficiently.

Further, the tablet will not degrade until it reacts completely to use up solid components. The tablet continues to react while remaining on the bottom of a bathtub and is able to dissolve the thus generated carbon dioxide gas into liquid efficiently as much as possible, thereby maximizing the effects of the present invention.

The organic acid is at least citric acid, and may include succinic acid and malic acid. The organic acid which includes at least citric acid can be used to effect continuously and effectively neutralization reactions in the tablet and also to generate fine bubbles. Therefore, the organic acid is able to provide, as a preferable compound, the effects of the present invention more remarkably.

According to a preferred embodiment of the present invention, citric acid and an anhydride such as anhydrous sodium carbonate are specified for the quantities in relation to a granulated substance A of bicarbonate. And, a tablet is prepared which is higher in hardness than a certain hardness and larger in size than a certain size, by which neutralization reactions can be effected at a maximum efficiency in the tablet and an aqueous solution after dissolution can be made neutral or weakly alkaline. As a result, it is possible to obtain the above-described maximum effects of the present invention.

Then, where bicarbonate is granulated by using a fluidized bed to obtain the granulated substance A, a mechanical fluidized-bed granulating machine which does not substantially utilize air for agitation can be used to efficiently enhance reactions in the tablet. In the mechanical agitation-type fluidized bed, upon agitation, no air is used for fluidization but a mechanical blade such as a propeller is used to fluidize powder. Thus, the mechanical agitation-type fluidized bed will not absorb moisture coming from wet air during granulation and is able to realize a vacuum during granulation by using a vacuum pump and carry out granulation, with polyethylene glycol decreased in quantity. The fluidized bed is able to exhibit the effects of making bubbles extremely small in diameter, while further activating neutralization reactions. Therefore, it is used preferably.

The mechanical fluidized-bed granulating machine which does not substantially utilize air for agitation is a mixer in which a plow-like shovel is installed on a horizontal-type drum to cause centrifugation, diffusion and vortex flow actions, thereby effecting three-dimensional fluidization. The machine is marketed by Gebruder Lödige Maschinenbau GmbH in Germany and Matsuzaka Engineering Co., Ltd. in Japan.

It is more preferable that the granulating machine is provided with a vacuum pump for reduction in pressure. That is, it is preferable that the machine is operated so as to reduce pressure on cooling and remove moisture as much as possible, thereby enhancing the effects of the present invention. It is more preferable that the granulating machine is provided with a chopper for preventing granulated grains from becoming coarse particles on cooling. That is, the chopper is actuated on cooling to make particles uniform, thereby exhibiting the effects of the present invention, that is, bubbles of carbon dioxide gas are made smaller in diameter than a microsize. And, this is a more preferable granulation method.

In the present invention, the most preferable tablet manufacturing method is such that sodium bicarbonate is granulated together with polyethylene glycol by using a fluidized-bed granulating machine on the basis of a mechanical agitation method, an organic acid, anhydrous sodium carbonate and polyethylene glycol are added at certain ratios to the thus prepared granulated substance and mixed, thereafter, they are subjected to compression molding at a high pressure, thereby giving a tablet which is higher in hardness than a certain hardness and larger in size than a certain size. Thus, the tablet is able to exhibit the effects of the present invention greatly.

As a matter of course, a mixture which is mainly composed of organic acid is granulated together with polyethylene glycol, which is only mixed with polyethylene glycol without granulation of bicarbonate, and a resultant thereof is then mixed with an organic-acid granulated substance and subjected to compression molding to prepare a tablet. This is also a preferable tablet manufacturing method in view of a relatively smaller quantity of compounds used for granulation and steps involved therein. In any case, in view of cost, it is desirable that a tablet is manufactured by procedures in which one of bicarbonate and an organic acid is granulated and the other is only mixed. In order that neutralization reactions occurring in the tablet of the present invention are kept for a prolonged period of time to facilitate the dissolution of carbon dioxide gas, both of bicarbonate and the organic acid salt are used by being mixed with polyethylene glycol or being coated. And, this is a preferable tablet manufacturing method.

In the present invention, PEG with the average molecular weight of 4000 to 8000 is preferably used in exhibiting the effects of the present invention. When a compression molding tablet machine such as a rotary-type tablet machine is used, PEG with the average molecular weight of about 6000 is able to provide preferable granulating results such as improvement in molding stability, rod attachment resistance, capping and tablet molding velocity. Therefore, where the tablet is dissolved in hot water, carbon dioxide gas components can be dissolved in a maximum quantity so as to give bicarbonate ions, and the tablet is increased in hardness, thickness and diameter, thereby, exhibiting more remarkable effects of the present invention.

A ratio of polyethylene glycol in relation to the granulated substance A of bicarbonate (sodium hydrogen carbonate or potassium hydrogen carbonate) or the mixture A of 100 parts by mass is preferably from 1:100 to 1:5 and in particular, preferably from 1:100 to 1:10. Where the ratio of PEG is smaller than the above-described ratio, bubbles of carbon dioxide gas are increased in diameter and decreased in bubble generation time, thus resulting in a possibility in which carbon dioxide gas components dissolved in hot water cannot be facilitated. On the other hand, where the ratio of polyethylene glycol is greater than the above-described ratio, generation of bubbles may be suppressed to reduce the quantity of carbon dioxide gas which is also dissolved.

Further, in the present invention, in a step where the granulated substance A of bicarbonate or the mixture A of PEG has been obtained and thereafter an organic acid, an organic-acid granulated substance B or a PEG organic acid mixture is added, an anhydride such as anhydrous sodium carbonate or anhydrous potassium carbonate is added, thus making it possible to exhibit the effect of the present invention more remarkably. It has been found that effects are obtained that, with bubbles of carbon dioxide gas made optimally small in diameter, the bubbles are generated in a greater quantity to keep reactions for a prolonged period of time.

Further, addition of anhydrous sodium carbonate provides more preferable effects of the present invention. And, anhydrous sodium carbonate is a compound that provides the above effects.

Still further, in the present invention, where compression molding is conducted by procedures in which polyethylene glycol is only added to the granulated substance A and an organic acid, without granulation of the organic acid, it has been found that microsize bubbles of the present invention can be generated for a prolonged period of time to facilitate dissolution of carbon dioxide gas components in hot water. Thereby, it is possible to obtain a favorable tablet. In this case, not only can steps be omitted to a great extent but also costs can be reduced, and therefore, this is a desirable manufacturing method.

On the other hand, where compression molding is conducted by procedures in which an organic acid is granulated by using PEG and bicarbonate is only mixed with PEG at a certain temperature, it is also possible to generate microsize bubbles of the present invention for a prolonged period of time and to maximize carbon dioxide gas components dissolved in hot water. Further, not only can steps be omitted to a great extent but also costs can be reduced. Thus, this is also found to be a desirable manufacturing method.

In the above-described manufacturing method, it is preferable that polyethylene glycol is used together with an organic acid in a range of 5 to 15 parts by mass in relation to the organic acid of 100 parts by mass.

In exhibiting the effects of the present invention, it is desirable that the organic acid, the organic acid mixture B or the organic acid granulated substance B is added in a ratio of 1:10 to 1:3 in relation to the bicarbonate granulated substance A or the PEG mixture A.

The effects of the present invention can be obtained upon addition of an anhydride, even if the organic acid is not granulated in particular. It is, however, more preferable that the organic acid is added together with polyethylene glycol or made into the PEG granulated substance B, mixed with the granulated substance A and subjected to compression molding. Thereby, it is possible to manufacture a tablet having preferable neutralization reactions.

Further, in exhibiting the effects of the present invention, it is preferable that an anhydride is added at any step before compression molding is conducted such as a step in which the granulated substance A or the mixture A is prepared and a step in which the granulated substance A is mixed with the organic acid or the granulated substance B.

Where the anhydride is added in an excessively large quantity, bubbles are generated in a small quantity. On the other hand, where the anhydride is added in an excessively small quantity, carbon dioxide gas is intensively generated in a bath, which is not preferable.

Further, in the present invention, one or two or more of anhydrides selected from anhydrous sodium carbonate and anhydrous potassium carbonate are used as an anhydride only in a range of 1:100 to 1:10 in relation to bicarbonate. Thereby, the effects of the present invention can be preferably exhibited.

In particular, an anhydride which exhibits maximum effects of the present invention includes anhydrous sodium carbonate.

In the present invention, it is possible to use a mold release agent when the tablet is molded. As the mold release agent, sucrose or magnesium stearate is generally used. As a compound which is in particular preferable in the present invention, at least any one of sodium n-(normal) octane sulfonate, sodium lauryl sulfonate, sodium lauroyl sarcosinate and myristoyl sodium methylalanine is contained, thus making it possible to manufacture the tablet according to the present invention stably and continuously by compression molding at a high speed, and this is preferable. Further, the above-described mold release agents are used most preferably in view of the fact that where the tablet according to the present invention is dissolved in hot water, microsize bubbles are generated and hot water after dissolution is kept clear. In addition, there are no particular restrictions on the quantity of the mold release agent added in the present invention, as long as it is added in a publicly known quantity.

In the present invention, it is acceptable that components (additives) other than main components are mixed whenever necessary. The main components include sodium hydrogen carbonate or potassium hydrogen carbonate as bicarbonate and citric acid, succinic acid, fumaric acid and malic acid as an organic acid. The most preferable organic acid is citric acid, and the maximum effects of the present invention can be obtained upon addition of citric acid. Other additives include health-related components such as hyaluronic acid, flavors, pigments, surface-active agents and anhydrides such as sodium carbonate, whenever necessary.

Anhydrides such as sodium carbonate, flavors, pigments, surface-active agents and polyethylene glycol may be used as a desirable additive in the organic acid, the organic acid mixture B or the granulated substance B.

Any publicly known compression molding machine can be used without special restrictions in conducting compression molding for manufacturing the tablet. For example, a hydraulic pressing machine, a single tablet machine, a rotary-type tablet machine and a briquetting machine, may be used. A rod used in the tablet machine is preferably 7mm or more in diameter where the rod is formed in a circular shape. Where the rod is formed in a triangle or rectangular shape, the rod is preferably 7mm or more in diameter which is calculated on conversion to a circular rod. This also applies to the thickness of the rod. Where a circular tablet is manufactured, the diameter of the tablet is 7mm or more and more desirably 10mm or more, and the thickness thereof is also 7mm or more and more preferably 10mm or more. Where the tablet is formed in a triangle or rectangular shape, it is more preferable that the tablet is 7mm or more upon conversion to a circular tablet both in diameter and thickness.

As described above, the tablet is not necessarily formed in a circular shape having a flat face. The tablet may be formed in an oval or spherical shape as long as it is a solid substance with a diameter of 7mm or more, with no restrictions on its shape.

The tablet may be formed in any shape, as long as it is a hard solid body which is larger in size than a certain size, able to generate microsize bubbles slowly inside the solid body and to dissolve carbon dioxide gas more effectively in liquid. The tablet is 25kg or more in hardness and 7mm or more in diameter and thickness. The more, the tablet is increased in hardness, the more effectively, the tablet generates carbon dioxide gas. Thereby, carbon dioxide gas is dissolved into the liquid effectively and bubbles are decreased in diameter, thereby providing preferable results.

Hereinafter, a description will be given of hardness of the tablet in the present invention.

In order to implement the present invention, hardness of the tablet was measured by using a Micro-Vickers hardness tester, Mitsutoyo HM-221, one of the hardness testers used in examples of many patent specifications.

In the present invention, Vickers hardness is a value obtained by measurements performed four times and expressed by HV and unit of kg/mm₂.

It was found that the measurement was made four times to average the results thereof, thus making it possible to make a substantially correct measurement. In examples to be described later, a mean value obtained by four time measurements is used.

### (Measured by using the tester at Jonan Branch of the Tokyo Metropolitan Industrial Technology Research Institute)

The diameter of bubbles of carbon dioxide gas generated in liquid, that is, a preferable condition of the tablet of the present invention, was macroscopically observed to find that the bubbles were 5mm or less in diameter and not joined together but generated uniformly. In order that the tablet continued to react, while remaining at the bottom until termination of neutralization reactions to dissolve the tablet completely, thereby efficiently dissolving carbon dioxide gas into the liquid, 12 different samples of 7 lots of the tablets manufactured on a trial basis were used to measure the Vickers hardness. The measurement was made four times to obtain a mean value, thus making it possible to disregard a variation in measured values of the Vickers hardness. It was confirmed that the tablets were able to meet requirements of the present invention that the surface mean Vickers hardness was 25kg or more.

Measurement was also made for hardness in terms of destruction strength of the tablets in a diameter direction.

In the above-described measurement, the tablets were measured for destruction strength. As a method for measuring the hardness in the diameter direction, a digital tablet hardness tester, New Speed Checker TS75NL made by Okada Seiko Co., Ltd. was used to measure the hardness of the tablets [kgf] four times. In this case as well, the hardness was reproducible and no great variation in values was found. However, the method was not reasonable in expressing the effects of the present invention. In the examples shown below, measurement was made for both Vickers hardness and hardness of expressing destruction hardness in the diameter direction. This measurement was well correlated to the effects of the present invention and able to sufficiently explain the effects of the present invention. Thus, a value measured for the hardness in the diameter direction was not adopted but only Vickers hardness was adopted.

In the present invention, it is desirable that compounds other than the compounds of the present invention are not added as much as possible. It is, however, possible to add one or two or more of different acidic components, alkaline components, flavors or turbid spring components, whenever necessary.

In the present invention, where a quantity of organic acid components in relation to bicarbonate exceeds a range specified in the present invention, the bubbles are increased in diameter, and reactions become intense and terminate in a short period of time. Further, where a quantity of organic acid components in relation to bicarbonate is excessively small, neutralization reactions will not take place efficiently to generate carbon dioxide gas in a small quantity, thereby exhibiting no effects of the present invention.

Further, where a quantity of sodium carbonate which is an anhydride is excessively small, polyethylene glycol must be used in an increased quantity. Otherwise, neutralization reactions would become too intense and bubbles would be increased in diameter to spoil the effect of the present invention. Still further, where a quantity of polyethylene glycol is excessively large or small in relation to bicarbonate or an organic acid, neutralization reactions will not take place uniformly and continuously or bubbles are not constant in diameter. Thus, it is not possible to obtain desirable effects of the present invention.

As described above, a desirable embodiment is that components required by the present invention are added in a ratio specified in the present invention, a pH adjusting agent is added so as to obtain the sufficient effects of the present invention, and an aqueous solution after dissolution of the tablet is kept in a pH range specified in the present invention.

### [Example-1]

Hereinafter, a detailed description will be given of the present invention by referring to examples, to which the present invention shall not be, however, limited.

### Granulation of comparative raw materials

### Operation-1

The following operation was conducted by using a fluidized-bed granulating machine GPCG-300CT made by Powrex Corporation.

Sodium hydrogen carbonate, 460kg, was added to the fluidized-bed granulating machine GPCG-300 CT made by Powrex Corporation installed in a granulation chamber air-conditioned so as to give 23°C and 60%RH and then heated. When a powder thereof reached a temperature of 68°C, PEG #6000 was fed at a quantity of 80kg, and a resultant was granulated by being heated up to 72°C. After the granulation was terminated, flowing air was set at 20°C to cool the powder. When the powder reached a temperature of about 35°C, a granulated substance was taken into a sealed container outside to terminate the operation, and a granulated substance A01 was obtained.

The following operation was conducted in a similar manner by using the fluidized-bed granulating machine GPCG-300CT made by Powrex Corporation.

Anhydrous citric acid and PEG #6000 were fed at the respective quantities of 380kg and 40kg into the fluidized-bed granulating machine GPCG-300 CT made by Powrex Corporation installed in the granulation chamber air-conditioned so as to give 23°C and 60%RH. Granulation was conducted at temperatures from 45°C to 69°C and after termination thereof, air was set at 20°C to cool the powder. When the powder reached a temperature of about 35°C, granulation was terminated and the powder was discharged into a sealed container and stored. Then, a granulated substance B01 was obtained.

### Operation-2

Anhydrous sodium carbonate, 460kg, was added to a modified type of Lödige mixer VT1200 made by Matsuzaka Engineering Co., Ltd. When a powder thereof reached a temperature of 45°C, polyethylene glycol #6000 was added in a quantity of 80kg and a resultant was granulated. When the powder reached a temperature of 70°C, granulation was terminated to cool the resultant with cold water at 20°C, and a granulated substance A02 was obtained.

Further, citric acid and PEG #6000 were added at the respective quantities of 380kg and 40kg to the modified type of the Lödige mixer VT1200 made by Matsuzaka Engineering Co., Ltd. from the time when a powder of the citric acid reached a temperature of 45°C and a resultant was granulated. When the powder reached a temperature of 69°C, granulation was stopped to cool indirectly the resultant with cold water kept at 20°C, and a granulated substance B02 was obtained.

The Lödige mixer made by Matsuzaka Engineering Co., Ltd. was used commonly hereinafter and the following method was conducted. That is, the modified type of the Lödige mixer VT1200 made by Matsuzaka Engineering Co., Ltd. was installed in the granulation chamber air-conditioned to give 23°C and 60% RH. Sodium hydrogen carbonate was fed in a specified quantity and agitated at the rotation frequency of 115rpm, and hot water kept at a specified temperature was circulated in a jacket to raise the temperature of the powder. When the powder reached a specified temperature, PEG #6000 was fed in a specified quantity. When the powder reached the specified temperature and after the elapse of certain time, granulation was terminated. Water in the jacket was decreased in temperature to replace the hot water and also cooled under a reduced pressure of 10 torr. When the powder reached a temperature of about 35°C, the powder was discharged through a discharge port on the bottom, stored in a sealed container, and a granulated substance was obtained.

In addition, detailed operation for obtaining the granulated substance is the same or similar to the above-described method and, therefore, the operation may be omitted from time to time.

### Granulation of raw materials in an example of the present invention Operation-3

The following operation was conducted by using the fluidized-bed granulating machine GPCG-300CT made by Powrex Corporation.

Sodium hydrogen carbonate, 460kg, was fed into the fluidized-bed granulating machine GPCG-300CT made by Powrex Corporation installed in the granulation chamber air-conditioned to give 23°C and 60%RH. When a powder thereof reached a temperature of 53°C, PEG #6000 was added in a quantity of 32kg. Heating was stopped when the temperature reached 63°C and, thereafter, the powder was started to be cooled by the flowing air set at 20°C. When the powder reached a temperature of about 35°C, granulation was terminated, and the powder was discharged into a sealed container, stored and a granulated substance A3 was obtained.

The following operation was conducted in a similar manner by using the fluidized-bed granulating machine GPCG-300CT made by Powrex Corporation.

Anhydrous citric acid and PEG #6000 were fed at the respective quantities of 60kg and 12kg to the fluidized-bed granulating machine GPCG-300CT made by Powrex Corporation installed in the granulation chamber air-conditioned to give 23°C and 60%RH. Flowing air set at 63°C was used to fluidize and granulate a powder thereof. After granulation was completed, the flowing air was set at 15°C to cool the powder. When the powder reached a temperature of about 35°C, granulation was terminated. Then, the powder was discharged into a sealed container, stored and a granulated substance B3 was obtained.

Sodium hydrogen carbonate and polyethylene glycol #6000 were added at the respective quantities of 460kg and 32kg to the modified type of the Lödige mixer VT1200 made by Matsuzaka Engineering Co., Ltd. and a resultant powder was granulated at 62°C. After granulation was terminated, cold water was used to cool indirectly the powder and a granulated substance A4 was obtained.

In a similar manner, citric acid and PEG #6000 were added at the respective quantities of 60kg and 10kg to the modified type of the Lödige mixer VT1200 made by Matsuzaka Engineering Co., Ltd. A resultant powder was granulated at 62°C and after granulation was terminated, the powder was cooled and a granulated substance B4 was obtained.

### Operation-4

### Granulation of raw materials in an example of the present invention

The following operation was conducted by using the fluidized-bed granulating machine GPCG-300CT made by Powrex Corporation.

Sodium hydrogen carbonate, PEG #6000 and anhydrous sodium carbonate were mixed at the respective quantities of 460kg, 35kg and 12kg in the fluidized-bed granulating machine GPCG-300CT made by Powrex Corporation installed in the granulation chamber air-conditioned to give 23°C and 60%RH, and a resultant powder was granulated at 51°C. Thereafter, flowing air set at 20°C was used to cool the powder. When the powder reached a temperature of about 35°C, granulation was terminated, the powder was discharged into a sealed container, stored and a granulated substance A5 was obtained.

The following operation was conducted in a similar manner by using the fluidized-bed granulating machine GPCG-300CT made by Powrex Corporation.

Anhydrous citric acid, PEG #6000 and anhydrous sodium carbonate were mixed at the respective quantities of 70kg, 12kg and 8kg in the fluidized-bed granulating machine GPCG-300CT made by Powrex Corporation installed in the granulation chamber air-conditioned to give 23°C and 60%RH, and a resultant powder was granulated at 62°C. After granulation was completed, flowing air was set at 15°C to cool the powder. When the powder reached a temperature of about 35°C, granulation was terminated, the powder was discharged into a sealed container, stored and a granulated substance B5 was obtained.

### Operation-5

### Granulation of raw materials in an example of the present invention

Sodium hydrogen carbonate, sodium carbonate and polyethylene glycol #6000 were added in the respective quantities of 460kg, 12kg and 20kg to the modified type of the Lödige mixer VT1200 made by Matsuzaka Engineering Co., Ltd. and a resultant powder was granulated at 60°C. After granulation was terminated, the powder was cooled and a granulated substance A6 was obtained.

Citric acid, polyethylene glycol #6000 and anhydrous sodium carbonate were mixed similarly in the respective quantities of 85kg, 8 kg and 9kg in the modified type of Lödige mixer VT1200 made by the Matsuzaka Engineering Co., Ltd. and a resultant powder was granulated at 53°C. After granulation was terminated, the powder was cooled and a granulated substance B6 was obtained.

### Operation-6

### Preparation of comparative tablets

### Comparative sample 1

Polyethylene glycol #6000, 6kg, and magnesium stearate, 1.5kg, were fed into the granulated substance A01, 540kg, and the granulated substance B01, 320kg, and they were mixed. Thereafter, an oil press type (manual tablet machine) made by Applied Power Industries [Applied Powder Japan, Ltd.] (formerly: Toyo Hydraulic Machine, Ltd.: model: SPLF-SPF-393) was used to apply a load of 1 ton, thereby preparing a tablet J01 with a diameter of 3mm and a thickness of 3mm.

### Comparative sample 2

Polyethylene glycol #6000, 6kg, and magnesium stearate, 1.5kg, were fed into the granulated substance A02, 540kg, and the granulated substance B02, 320kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 1 ton, thereby preparing a tablet J02 with a diameter of 30mm and a thickness of 15mm.

### Comparative sample 3

The granulated substance B02, 320kg, polyethylene glycol #6000, 6kg, and magnesium stearate, 1.5kg, were fed into the granulated substance A01, 540kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 2 tons, thereby preparing a tablet J03 with a diameter of 7mm and a thickness of 4mm.

### Comparative sample 4

The granulated substance B01, 320kg, polyethylene glycol #6000, 6kg and magnesium stearate, 1.5kg, were fed into the granulated substance A02, 540kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 2 tons, thereby preparing a tablet J04 with a diameter of 30mm and a thickness of 4mm.

### Comparative sample 5

Citric acid, 80kg, polyethylene glycol #6000, 16kg, and magnesium stearate, 1.5kg, were fed into the granulated substance A01, 540kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 2 tons, thereby preparing a tablet J05 with a diameter of 9mm and a thickness of 5mm.

### Comparative sample 6

Citric acid, 80kg, polyethylene glycol #6000, 16kg, and magnesium stearate, 1.5kg, were fed into the granulated substance A02, 540kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 1 ton, thereby preparing a tablet J06 with a diameter of 8mm and a thickness of 8mm.

### Operation-7

### Sample 1 (reference example)

Citric acid, 60kg, polyethylene glycol #6000, 16kg, anhydrous sodium carbonate, 20kg, and n-octane sulfonate, 1.5kg, were fed into the granulated substance A03, 500kg, and they were agitated and mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 4 tons, thereby preparing a tablet J1 with a diameter of 20mm and a thickness of 15mm.

### Sample 2 of the present invention

Citric acid, 60kg, anhydrous sodium carbonate, 35kg, polyethylene glycol #6000, 8kg, and n-octane sulfonate, 1.5kg, were fed into the granulated substance A4, 500kg, and they were agitated at the rotation frequency of 115rpm and mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 12 tons, thereby preparing a tablet J2 with a diameter of 30mm and a thickness of 15mm.

### Sample 3 of the present invention

Citric acid, 60 kg, polyethylene glycol #6000, 10kg, n-octane sulfonate, 1.0kg and sodium lauryl sulfonate, 1kg, were fed into the granulated substance A5, 500kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 9 tons, thereby preparing a tablet J3 with a diameter of 60mm and a thickness of 20mm.

### Sample 4 of the present invention

Citric acid, 100kg, anhydrous sodium carbonate, 8kg, polyethylene glycol #6000, 8kg, and sodium n-octane sulfonate, 1.5kg, were fed into the granulated substance A3, 500kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 9 tons, thereby preparing a tablet J4 with a diameter of 30mm and a thickness of 15mm.

### Sample 5 (reference example)

Succinic acid, 100kg, was added to the granulated substance A4, 500kg, and anhydrous sodium carbonate, 23kg, polyethylene glycol, 8kg, and sodium n-octane sulfonate, 1.5kg, were added thereto. The oil press type (manual tablet machine) made by [Applied Powder Japan, Ltd.] (formerly: Toyo Hydraulic Machine, Ltd.: model: SPLF-SPF-393) was used to apply a load of 9 tons, thereby preparing a tablet J5 with a diameter of 30mm and a thickness of 15mm.

### Sample 6 (reference example)

Fumaric acid, 100kg, was added to the granulated substance A4, 500kg, and anhydrous sodium carbonate, 23kg, polyethylene glycol, 8kg, and sodium n-octane sulfonate, 1.5kg were added thereto. The oil press type (manual tablet machine) made by [Applied Powder Japan, Ltd.] (formerly: Toyo Hydraulic Machine, Ltd.: model: SPLF-SPF-393) was used to apply a load of 9 tons, thereby preparing a tablet J6 with a diameter of 30mm and a thickness of 15mm.

### Sample 7 (reference example)

Malic acid, 100 kg, was added to the granulated substance A4, 500kg, and anhydrous sodium carbonate, 23kg, polyethylene glycol, 8kg, and sodium n-octane sulfonate, 1.5kg, were added thereto. The oil press type (manual tablet machine) made by Applied Power Industries [Applied Powder Japan, Ltd.] (formerly: Toyo Hydraulic Machine, Ltd.: model: SPLF-SPF-393) was used to apply a load of 9 tons, thereby preparing a tablet J7 with a diameter of 30mm and a thickness of 15mm.

### Sample 8 of the present invention

Citric acid, 80kg, anhydrous potassium carbonate, polyethylene glycol #6000, 6kg, and n-octane sulfonate, 1.5kg, were fed into the granulated substance A4, 500kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 9 tons, thereby preparing a tablet J8 with a diameter of 30mm and a thickness of 15mm.

### Sample 9 of the present invention

The granulated substance B6, 80kg, polyethylene glycol #6000, 7kg, and sodium lauryl sulfate, 1.5kg, were fed into the granulated substance A4, 500kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 9 tons, thereby preparing a tablet J9 with a diameter of 30mm and a thickness of 15mm.

### Sample 10 of the present invention

Citric acid, 60kg, polyethylene glycol #6000, 12kg, sodium carbonate, 20kg, and sodium n-octane sulfonate, 1.5kg, were fed into the granulated substance A4, 500kg, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 9 tons, thereby preparing a tablet J10 with a diameter of 30mm and a thickness of 15mm.

### Sample 11 of the present invention

Citric acid, 60kg, anhydrous potassium carbonate, 9kg, polyethylene glycol #6000, 10kg, and sodium n-octane sulfonate 1.5kg, were fed into the granulated substance A4, 500kg, and they were fixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 9 tons, thereby preparing a tablet J11 with a diameter of 30mm and a thickness of 15mm.

### Operation-8

### Evaluation procedures of tablet of example-1

### (1) Measurement of hardness, measurement of Vickers hardness

The Micro-Vickers hardness tester Mitsutoyo HM-221 was used to measure the hardness of the tablet (HV, kg/mm₂) four times, and a mean value thereof was shown in Table 1 shown below. (2) Evaluation of bubble generation state on dissolution of tablet

Water at a temperature of about 35°C was placed into a glass cylinder with a diameter of 70mm and the height of 400mm up to the height of 250mm. A sample of each tablet which was adjusted to give about 15g was placed as it is in such a manner that the tablet would not be changed in its shape as much as possible. Observations were made for a state of bubble generation and a state of rising bubbles on the basis of the following criteria. Complete dissolution time of each tablet was measured and recorded, and the results are shown in Table 1 shown below.
●: Bubbles with a diameter of about 10mm to 15mm are hardly found. Bubbles with a diameter of about 2mm to 6mm rise uniformly but float up to the surface of liquid in a small number. The number of these bubbles is decreased on the way. A state that carbon dioxide gas is dissolved is found.
○: Bubbles with a diameter of about 10mm to 15mm account for 20% or less, and the bubbles are mostly generated as being fine and uniform in size. A state that the bubbles are dissolved while rising is found.
Δ: Bubbles are small and uniform in size but generated excessively slowly, with no force being found, and dissolved in a small quantity. Or the generated bubbles are joined together and increased in size. The bubbles rise and escape into the air at a larger percentage.
×: Large bubbles with a diameter of about 10mm to 15mm account for 40% or more. The bubbles are intensively generated, mostly joined together and increased in size. The bubbles rise up to the surface of liquid quickly, and carbon dioxide gas escapes outside liquid. Or, a state that reactions take place excessively slowly and bubbles are joined together and escape into the air is found.
××: Bubbles are generated intensively and mostly escape from the surface of liquid into the air and generation of bubbles will end in a few minutes. Or, bubbles are hardly generated or no neutralization reactions take place, and dissolution time is also long. A state that carbon dioxide gas is much less likely to be dissolved in water is inferable. (3) Measurement of complete dissolution time of tablet

Time necessary for dissolving 90% or more of a tablet (macroscopic observation) after the tablet was placed into the above-described glass cylinder, was recorded.

### (4) Warming effects at the time of bathing (temperatures were measured on the surface of the skin when a subject took a foot bath)

In a room kept at 24°C, both legs of the subject were submerged for 15 minutes into a foot bath constantly kept at 38°C. A thermographic camera TVS500IS was used to photograph the surface of the legs after one hour in each of three subjects. Warming continuity on the surface of the skin was evaluated on the basis of criteria shown below. The results are shown in Table 1.
• Red color is found all over an image photographed by the thermographic camera after one hour, and the body is also sufficiently warmed.
○ Yellow color is found on an image photographed by the thermographic camera after one hour, and warming effects are obtained.
× Blue color is found mostly on an image photographed by the thermographic camera after one hour, and warming effects are similar to those of ordinary hot water.

**[Table 1]**

| Samples | (1) Vickers hardness, kg | (2) pH after dissolution of 15g tablet /10L | (3) Distribution of generation of bubbles depending on diameter | (4) Complete dissolution time, sec | (5) Warming sensation of the body as shown in thermogram |
|---|---|---|---|---|---|
| J01 (comparison) | 3.3 | 4.6 | ×× | 123 | × |
| J02 (comparison) | 4.3 | 4.7 | ×× | 120 | × |
| J03 (comparison) | 4.8 | 4.9 | ×× | 120 | × |
| J04 (comparison) | 4.7 | 4.7 | ×× | 130 | × |
| J05 (comparison) | 6.6 | 7.1 | × | 260 | × |
| J06 (comparison) | 7.8 | 7.2 | × | 280 | × |
| J1 (reference example) | 23 | 7.8 | ○ | 420 | ○ |
| J2 (the present invention) | 108 | 7.4 | ● | 480 | ● |
| J3 (the present invention) | 66 | 7.2 | ○ | 420 | ● |
| J4 (the present invention) | 116 | 7.8 | ● | 460 | ● |
| J5 (reference example) | 21 | 6.2 | Δ | 900 | ○ |
| J6 (reference example) | 23 | 6.1 | Δ | 870 | ○ |
| J7 (reference example) | 96 | 6.4 | Δ | 530 | ○ |
| J8 (the present invention) | 30 | 8.1 | ○ | 460 | ○ |
| J9 (the present invention) | 46 | 7.8 | ● | 450 | ● |
| J10 (the present invention) | 120 | 6.8 | ○ | 540 | ● |
| J11 (the present invention) | 76 | 7.1 | ● | 420 | ● |

As apparent from Table 1, where the tablet of the present invention is higher in hardness than a certain hardness, greater in diameter and thickness than a certain size and pH of hot water immediately after dissolution of the tablet is within a pH value specified in the present invention, it is found that working effects particular to the present invention are provided.

### [Example-2]

### Operation-9

Quantities of added tablet components and granulation conditions for giving an effective scope of the present invention were changed by the following operation. The operation was conducted as follows in a similar manner as performed in Example-1. Evaluation was made similarly as performed in the above-described operation-8. The results are shown below.

That is, the granulated substance A4 was added in a quantity of 500kg and citric acid was added by changing a quantity thereof as follows:
J12: citric acid (300kg) (reference example)
J13: citric acid (260kg) (reference example)
J14: citric acid (150kg)
J15: citric acid (100kg)
J16: citric acid (60kg)
J17: citric acid (10kg) (reference example)
J18: citric acid (5kg) (reference example)

Anhydrous sodium carbonate, 9kg, polyethylene glycol #6000, 10kg, and sodium n-octane sulfonate, 1.5kg, were fed to each of the above mixtures, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to apply a load of 8 tons, thereby preparing the tablets J12 to J18 with a diameter of 30mm and a thickness of 15mm. Evaluation was made by the above-described operation-8. The results are shown below.

**[Table 2]**

| Samples | (1) Vickers hardness, kg | (2) pH | (3) Distribution of generation of bubbles depending on diameter | (4) Complete dissolution time, sec | (5) Warming sensation of the body as shown in thermogram |
|---|---|---|---|---|---|
| J12: citric acid (300kg) (reference example) | 16 | 4.3 | ×× | 120 | × |
| J13: citric acid (260kg) (reference example) | 16 | 5.4 | ×× | 230 | × |
| J14: citric acid (150kg) | 46 | 6.2 | ○ | 280 | ○ |
| J15: citric acid (100kg) | 89 | 7.2 | ● | 450 | ● |
| J16: citric acid (60kg) | 108 | 7.8 | ● | 430 | ● |
| J17: citric acid (10kg) (reference example) | 32 | 8.4 | ○ | 780 | ○ |
| J18: citric acid (5kg) (reference example) | 32 | 9.8 | × | 890 | × |

The above-described results have revealed that there is an optimal range of citric acid (an organic acid) added to bicarbonate and the effects of the present invention are exhibited more favorably not only by the hardness of the tablet and a pH value after dissolution but also by an optimal quantity of organic acid in relation to bicarbonate.

### [Example-3]

### Operation-10

Citric acid, 80kg, and polyethylene glycol #6000, 15kg, were added to the granulated substance A4, 500kg, and sodium n-octane sulfonate, 1.5kg, was added thereto and they were mixed. Thereafter, to each of the mixtures divided into a certain quantity, sodium carbonate was added by changing a quantity thereof. The oil press type (manual tablet machine) made by Applied Power Industries [Applied Powder Japan, Ltd.] (formerly: Toyo Hydraulic Machine, Ltd.: model: SPLF-SPF-393) was used to apply a load of 8 tons to the mixtures, thereby preparing the tablets with a diameter of 30mm and a thickness of 15mm. From the tablets, those in which a pH value immediately after dissolution was 4.5 for J24 (reference example), 5.6 for J25, 6.2 for J26, 7.2 for J27, 7.8 for J28, 8.4 for J29, and 9.5 for J30 (reference example), were selected. Evaluation was made similarly as performed in Example 3, and the results are shown in the table shown below.

**[Table 3]**

| Samples | (1) Vickers hardness, kg | (2) pH | (3) Distribution of generation of bubbles depending on diameter | (4) Complete dissolution time, sec | (5) Warming sensation of the body as shown in thermogram |
|---|---|---|---|---|---|
| J24 (reference example) | 49 | 4.5 | ×× | 220 | × |
| J25 | 44 | 5.6 | ○ | 460 | ○ |
| J26 | 41 | 6.2 | ○ | 670 | ● |
| J27 | 54 | 7.2 | ● | 780 | ● |
| J28 | 38 | 7.8 | ● | 760 | ● |
| J29 | 61 | 8.4 | ○ | 840 | ○ |
| J30 (reference example) | 67 | 9.5 | × | 1230 | × |

The above-described results have revealed that preferable results are obtained when a pH value immediately after dissolution of the tablet is from 5.5 or more to 8.5 or less, and in particular, more preferable results are obtained when a pH value is from 6.2 or more to 8.0 or less.

### [Example-4]

In the above-described Example-1, the tablet J3 was prepared in the same way only except that n-octane sulfonate and sodium lauryl sulfonate (mold release agent) were replaced by sodium lauroyl sarcosinate (J19) or myristoyl sodium methylalanine (J20), both of them (J21), sucrose (J22) or magnesium stearate (J23) at an equivalent quantity. The tablets J19, J20 and J21 were able to keep clear hot water after dissolution as with J3. However, the tablets J22 and J23 showed turbidity of hot water.

### [Example-5]

### Operation-11

Citric acid, 70kg, and polyethylene glycol #6000, 15kg, were added to the granulated substance A4, 500kg, to which anhydrous sodium carbonate was added as follows by changing a quantity thereof:
J31: 100kg (reference example),
J32: 50kg (reference example),
J33: 30kg,
J34: 10kg,
J35: 2kg (reference example), and
J36: 0kg (reference example).

After they were mixed, the oil press type (manual tablet machine) made by Applied Power Industries [Applied Powder Japan, Ltd.] (formerly: Toyo Hydraulic Machine, Ltd.: model: SPLF-SPF-393) was used to apply a load of 8 tons to the mixtures, thereby preparing the tablets J31 to J36 with a diameter of 30mm and a thickness of 15mm.

**[Table 4]**

| Samples | (1) Vickers hardness, kg | (2) pH | (3) Distribution of generation of bubbles depending on diameter | (4) Complete dissolution time, sec | (5) Warming sensation of the body as shown in thermogram |
|---|---|---|---|---|---|
| J31: 100kg (reference example) | 14 | 9.8 | × | 280 | × |
| J32: 50kg (reference example) | 46 | 8.9 | × | 320 | × |
| J33: 30kg | 86 | 8.1 | ● | 420 | ● |
| J34: 10kg | 97 | 7.2 | ● | 460 | ● |
| J35: 2kg (reference example) | 62 | 6.3 | ○ ∼ Δ | 260 | ○ |
| J36: 0kg (reference example) | 46 | 5.7 | ○ ∼ Δ | 130 | ○ |

The above-described results have revealed that where anhydrous sodium carbonate (anhydride) is added in a range of 1:100 to 1:10 in relation to sodium hydrogen carbonate (bicarbonate), the effects of the present invention are exhibited more favorably.

### [Eexample-6]

### Operation-12

In manufacturing the granulated substance A4, sodium hydrogen carbonate, 460kg, was added thereto together with polyethylene glycol #6000 which was weighed so as to change the quantity as follows, and each of the thus prepared resultant mixtures was granulated by using the modified type of Lödige mixer VT1200 made by using Matsuzaka Engineering Co., Ltd. Polyethylene glycol was added to conduct granulation when the powder of sodium hydrogen carbonate reached a temperature of 53°C.
J37: 0kg (reference example)
J38: 1kg was fed (reference example)
J39: 10kg was fed
J40: 20kg was fed
J41: 40kg was fed
J42: 80kg was fed
J43: 120kg was fed (reference example)

The granulated substance A, each 500kg, citric acid, 80kg, anhydrous sodium carbonate, 10kg, and PEG #6000, 6kg, were added, and sodium n-octane sulfonate, 1.5kg, was fed thereto and they were mixed. Thereafter, the Tough Press Correct 1527HU (tablet machine) made by Kikusui Seisakusho Ltd. was used to apply a load of 6 tons to the mixtures, thereby preparing the tablets with a diameter of 30mm and a thickness of 15mm. Evaluation was made for the tablets similarly as performed in the operation-8. The results are shown in the table shown below.

**[Table 5]**

| Samples | (1) Vickers hardness, kg | (2) pH | (3) Distribution of generation of bubbles depending on diameter | (4) Complete dissolution time, sec | (5) Warming sensation of the body as shown in thermogram |
|---|---|---|---|---|---|
| J37: 0kg, no addition (reference example) | No granulation was provided to result in failure of evaluation | | | | |
| J38: 1kg was fed (reference example) | No granulation was provided to result in failure of evaluation | | | | |
| J39: 10kg was fed | 35 | 7.3 | ○ | 380 | ○ |
| J40: 20kg was fed | 98 | 7.3 | ● | 440 | ● |
| J41: 40kg was fed | 48 | 7.4 | ● | 480 | ● |
| J42: 80kg was fed | 86 | 7.4 | × | 1670 | ○ |
| J43: 120kg was fed (reference example) | No granulation was provided to result in failure of evaluation | | | | |

The above-described results have revealed that the effects of the present invention are obtained when polyethylene glycol is added in a range of 1:100 to 1:5 in relation to sodium hydrogen carbonate (bicarbonate), more preferably in a range of 1:100 to 1:10.

### [Example-7]

### Operation-13

Citric acid, 70kg was added to the granulated substance A4, 500kg, and polyethylene glycol, 15kg, was also added, and anhydrous sodium carbonate was added thereto in quantities shown below, J44 to J50, and they were mixed. Thereafter, sodium n-octane sulfonate, 15kg, was added. Then, the oil press type (manual tablet machine) made by Applied Power Industries [Applied Powder Japan, Ltd.] (formerly: Toyo Hydraulic Machine, Ltd.: model: SPLF-SPF-393) was used to change loads as follows, thereby preparing the tablets (J44 to J50) with a diameter of 30mm and a thickness of 12mm.
J44: 1 ton (reference example)
J45: 2 tons (reference example)
J46: 3 tons (reference example)
J47: 4 tons (reference example)
J48: 5 tons
J49: 8 tons
J50: 9 tons

Evaluation was made for each of the tablets according to the operation-8, and the results are shown in the table shown below.

**[Table 6]**

| Samples | (1) Vickers hardness, kg | (2) pH | (3) Distribution of generation of bubbles depending on diameter | (4) Complete dissolution time, sec | (5) Warming sensation of the body as shown in thermogram |
|---|---|---|---|---|---|
| J44: 1 ton (reference example) | 1.7 | 7.3 | × | 180 | × |
| J45: 2 tons (reference example) | 4.8 | 7.3 | × | 210 | × |
| J46: 3 tons (reference example) | 7.2 | 7.2 | × | 230 | × |
| J47: 4 tons (reference example) | 22 | 7.3 | ○ | 360 | ○ |
| J48: 5 tons | 30 | 7.3 | ● | 430 | ○ |
| J49: 8 tons | 48 | 7.3 | ● | 540 | ● |
| J50: 9 tons | 125 | 7.7 | ● | 670 | ● |

Table 6 has revealed that where the tablet has hardness of 25kg or more, the effects of the present invention are made maximum.

### [Example-8]

### Operation-14

Citric acid, 70kg, was added to the granulated substance A4, 500kg, and polyethylene glycol, 15kg, was also added thereto, and anhydrous sodium carbonate was added in quantities shown in Operation-13, J44 to J50, and they were mixed. Thereafter, the oil press type (manual tablet machine) made by Applied Power Industries [Applied Powder Japan, Ltd.] (formerly: Toyo Hydraulic Machine, Ltd.: model: SPLF-SPF-393) was used to change loads, with a mill rod also changed, thereby preparing the tablets (J51 to J57) in which a diameter and a thickness were changed as follows:
J51: 2 x 2mm (reference example)
J52: 4 x 4mm (reference example)
J53: 8 x 4mm (reference example)
J54: 15 x 8mm
J55: 20 x 15mm
J56: 30 x 15mm
J57: 60 x 25mm

Evaluation was made for each of the tablet samples according to the operation-8. The results are shown in the table shown below.

**[Table 7]**

| | (1) Vickers hardness, kg | (2) pH | (3) Distribution of generation of bubbles depending on diameter | (4) Complete dissolution time, sec | (5) Warming sensation of the body as shown in thermogram |
|---|---|---|---|---|---|
| J51: 2 x 2mm (reference example) | 3.7 | 6.8 | × | 180 | × |
| J52: 4 x 4mm (reference example) | 5.8 | 7.1 | × | 210 | × |
| J53: 8 x 4mm (reference example) | 10 | 6.8 | × | 230 | × |
| J54: 15 x 8mm | 29 | 7.0 | ○ | 380 | ○ |
| J55: 20 x 15mm | 76 | 7.0 | ● | 430 | ● |
| J56: 30 x 15mm | 98 | 7.1 | ● | 670 | ● |
| J57: 60 x 25mm | 95 | 6.8 | ● | 780 | ● |

Table 7 has revealed that where the tablet is 7mm or more both in diameter and thickness and able to meet the hardness and pH value specified in the present invention, the effects of the present invention are exhibited more favorably.

## Claims

1. A bathing tablet manufacturing method comprising compression molding an organic acid in the presence of polyethylene glycol, an anhydride, and a bicarbonate to achieve the bathing tablet,
wherein the organic acid is citric acid and optionally at least one selected from the group consisting of succinic acid, fumaric acid and malic acid, the anhydride is at least one selected from the group consisting of anhydrous sodium carbonate and anhydrous potassium carbonate, and the bicarbonate is at least one selected from the group consisting of sodium hydrogen carbonate and potassium hydrogen carbonate,
wherein the ratio of the organic acid to the bicarbonate is in the range of 1:10 to 1:3, the ratio of the anhydride to the bicarbonate is in the range of 1:100 to 1:10, and the ratio of the polyethylene glycol to the bicarbonate is in the range of 1:100 to 1:5,
wherein an aqueous solution immediately after dissolution of the bathing tablet has a pH of 5.5 to 8.5, and
wherein the bathing tablet has a hardness of 25 kg or more, a diameter of 7mm or more, and a thickness of 7mm or more.

2. The bathing tablet manufacturing method according to claim 1, in which at least one of the bicarbonate and the organic acid is a granulated substance which is mixed with polyethylene glycol and granulated by using a fluidized-bed granulating machine.

3. A bathing tablet, which is obtained by subjecting an organic acid to compression molding in the presence of polyethylene glycol, an anhydride, and a bicarbonate,
wherein the organic acid is citric acid and optionally at least one selected from the group consisting of succinic acid, fumaric acid and malic acid, the anhydride is at least one selected from the group consisting of anhydrous sodium carbonate and anhydrous potassium carbonate, and the bicarbonate is at least one selected from the group consisting of sodium hydrogen carbonate and potassium hydrogen carbonate,
wherein the ratio of the organic acid to the bicarbonate is in the range of 1:10 to 1:3, the ratio of the anhydride to the bicarbonate is in the range of 1:100 to 1:10, and the ratio of the polyethylene glycol to the bicarbonate is in the range of 1:100 to 1:5,
wherein an aqueous solution immediately after dissolution of the bathing tablet has a pH of 5.5 to 8.5, and
wherein the bathing tablet has a hardness of 25 kg or more, a diameter of 7mm or more, and a thickness of 7mm or more.

4. The bathing tablet according to claim 3, in which an aqueous solution immediately after dissolution of the bathing tablet is from 6.0 to 8.0 in pH.

5. The bathing tablet according to claim 3, which contains at least one of sodium n-(normal) octane sulfonate, sodium lauryl sulfonate, sodium lauroyl sarcosinate and myristoyl sodium methylalanine.

## Patentansprüche

1. Badetablettenherstellungsverfahren umfassend Formpressen einer organischen Säure in Gegenwart von Polyethylenglykol, einem Anhydrid und einem Bicarbonat, um die Badetablette zu gewinnen,
wobei die organische Säure Zitronensäure ist und wahlweise mindestens eine ist ausgewählt aus der Gruppe bestehend aus Bernsteinsäure, Fumarsäure und Apfelsäure, das Anhydrid mindestens eines ist ausgewählt aus der Gruppe bestehend aus wasserfreiem Natriumcarbonat und wasserfreiem Kaliumcarbonat und das Bicarbonat mindestens eines ist ausgewählt aus der Gruppe bestehend aus Natriumhydrogencarbonat und Kaliumhydrogencarbonat,
wobei das Verhältnis der organischen Säure zu dem Bicarbonat im Bereich von 1:10 bis 1:3 liegt, das Verhältnis des Anhydrids zu dem Bicarbonat im Bereich von 1:100 bis 1:10 liegt und das Verhältnis des Polyethylenglykols zu dem Bicarbonat im Bereich von 1:100 bis 1:5 liegt,
wobei eine wässrige Lösung unmittelbar nach Lösen der Badetablette einen pH-Wert von 5,5 bis 8,5 aufweist und
wobei die Badetablette eine Härte von 25 kg oder mehr, einen Durchmesser von 7 mm oder mehr und eine Dicke von 7 mm oder mehr aufweist.

2. Badetablettenherstellungsverfahren nach Anspruch 1, wobei mindestens eines von dem Bicarbonat und der organischen Säure eine granulierte Substanz ist, die mit Polyethylenglykol gemischt und unter Anwendung einer Wirbelbettgranuliermaschine granuliert wird.

3. Badetablette, welche erhalten wird, idem eine organische Säure Formpressen unterzogen wird in Gegenwart von Polyethylenglykol, einem Anhydrid und einem Bicarbonat,
wobei die organische Säure Zitronensäure ist und wahlweise mindestens eine ist ausgewählt aus der Gruppe bestehend aus Bernsteinsäure, Fumarsäure und Apfelsäure, das Anhydrid mindestens eines ist ausgewählt aus der Gruppe bestehend aus wasserfreiem Natriumcarbonat und wasserfreiem Kaliumcarbonat und das Bicarbonat mindestens eines ist ausgewählt aus der Gruppe bestehend aus Natriumhydrogencarbonat und Kaliumhydrogencarbonat,
wobei das Verhältnis der organischen Säure zu dem Bicarbonat im Bereich von 1:10 bis 1:3 liegt, das Verhältnis des Anhydrids zu dem Bicarbonat im Bereich von 1:100 bis 1:10 liegt und das Verhältnis des Polyethylenglykols zu dem Bicarbonat im Bereich von 1:100 bis 1:5 liegt,
wobei eine wässrige Lösung unmittelbar nach Lösen der Badetablette einen pH-Wert von 5,5 bis 8,5 aufweist und
wobei die Badetablette eine Härte von 25 kg oder mehr, einen Durchmesser von 7 mm oder mehr und eine Dicke von 7 mm oder mehr aufweist.

4. Badetablette nach Anspruch 3, wobei eine wässrige Lösung unmittelbar nach Lösen der Badetablette einen pH-Wert von 6,0 bis 8,0 aufweist.

5. Badetablette nach Anspruch 3, die mindestens eines von Natrium-n-(normal)octansulfonat, Natriumlaurylsulfonat, Natriumlauroylsarcosinat und Myristoylnatriummethylalanin enthält.

## Revendications

1. Procédé de fabrication d'un comprimé pour le bain comprenant le moulage par compression d'un acide organique en présence de polyéthylène glycol, d'un anhydride, et d'un bicarbonate pour obtenir le comprimé pour le bain,
l'acide organique étant l'acide citrique et optionnellement au moins l'un sélectionné dans le groupe constitué de l'acide succinique, de l'acide fumarique et de l'acide malique, l'anhydride étant au moins l'un sélectionné dans le groupe constitué du carbonate de sodium anhydre et du carbonate de potassium anhydre, et le bicarbonate étant au moins l'un sélectionné dans le groupe constitué de l'hydrogénocarbonate de sodium et de l'hydrogénocarbonate de potassium,
le rapport de l'acide organique au bicarbonate se situant dans la plage de 1:10 à 1:3, le rapport de l'anhydride au bicarbonate se situant dans la plage de 1:100 à 1:10, et le rapport du polyéthylène glycol au bicarbonate se situant dans la plage de 1:100 à 1:5,
une solution aqueuse immédiatement après la dissolution du comprimé pour le bain ayant un pH de 5,5 à 8,5, et
le comprimé pour le bain ayant une dureté de 25 kg ou plus, un diamètre de 7 mm ou plus, et une épaisseur de 7 mm ou plus.

2. Procédé de fabrication d'un comprimé pour le bain selon la revendication 1, dans lequel au moins l'un parmi du bicarbonate et de l'acide organique est une substance granulée qui est mélangée avec le polyéthylène glycol et qui est granulée en utilisant une machine de granulation sur lit fluidisé.

3. Comprimé pour le bain, qui est obtenu par soumission d'un acide organique au moulage par compression en la présence de polyéthylène glycol, d'un anhydride, et d'un bicarbonate,
l'acide organique étant l'acide citrique et optionnellement au moins l'un sélectionné dans le groupe constitué de l'acide succinique, l'acide fumarique et de l'acide malique, l'anhydride étant au moins l'un sélectionné dans le groupe constitué du carbonate de sodium anhydre et du carbonate de potassium anhydre, et le bicarbonate étant au moins l'un sélectionné dans le groupe constitué de l'hydrogénocarbonate de sodium et de l'hydrogénocarbonate de potassium,
le rapport de l'acide organique au bicarbonate se situant dans la plage de 1:10 à 1:3, le rapport de l'anhydride au bicarbonate se situant dans la plage de 1:100 à 1:10, et le rapport du polyéthylène glycol au bicarbonate se situant dans la plage de 1:100 à 1:5,
une solution aqueuse immédiatement après la dissolution du comprimé pour le bain ayant un pH de 5,5 à 8,5, et
le comprimé pour le bain ayant une dureté de 25 kg ou plus, un diamètre de 7 mm ou plus, et une épaisseur de 7 mm ou plus.

4. Comprimé pour le bain selon la revendication 3, dans lequel une solution aqueuse immédiatement après la dissolution du comprimé pour le bain a un pH de 6,0 à 8,0.

5. Comprimé pour le bain selon la revendication 3, qui contient au moins l'un parmi le *n*-(normal) octane sulfonate de sodium, le lauryl sulfonate de sodium, le lauroyl sarcosinate de sodium et la myristoyl sodium méthylalanine.
